# EUROPEAN PATENT APPLICATION

(11) **EP 1 553 772 A2**
(43) Date of publication of application: **13.07.2005**
(21) Application number: 04256852.7
(22) Date of filing: 05.11.2004
(51) Int. Cl.: H04N 7/00

(54) **Interactive system**

(30) Priority: 10.11.2003 GB 0326170
(71) Applicant: The Wandsworth Group Limited, Woking, Surrey GU21 5SE (GB)
(72) Inventor: Mockett, Richard John, Midhurst, West Sussex, GU29 0LA (US)
(74) Representative: Johnson, Terence Leslie

(57) **Abstract**

The invention relates to a digital system 1 for providing services to a patient in a health care environment, such as a hospital 2, comprising a means 3 to display data, means 4, 4' operable by a user to select desired data and activate the display means 3, and remote data store means such as servers 5 adapted to provide data digitally to the display means 3 when accessed by the user. The overall system 1 can provide patient access to entertainment, communication, and medical data relating to that patient and held by or on a hospital data base. The medical condition of each patient, in other words an inventory of medical data, is thus accessible.

## Description

The invention relates to an interactive system, particularly a system for providing services to a patient in a health care environment such as a hospital.

In the United Kingdom it is Government policy that patients in major hospitals shall have bedside telephones and televisions by 2004. Also, by then, patients should have access to electronic personal record systems and 75% of hospitals are to have electronic record systems. At present patient bedside services are available, for example analogue television, radio and telephone services have been provided, which can also be paid for by a card, such as a smartcard, payment system.

These prior systems unfortunately generally provide only a limited range of services, for example being limited to television, video, voice and audio services. Moreover, such systems have an over-dependence on use of the telephone network, which is often high-cost being provided at a premium rate, are expensive to upgrade and rely on multiple networks which are generally incompatible with hospital data systems, being usually dependent on separate co-ax and telephone networks to deliver patient services. Thus prior systems are expensive to use and provide limited benefits to patients and hospitals alike.

It is accordingly an object of the invention to seek to mitigate these disadvantages.

According to the invention there is provided a digital system for providing services to a patient in a health care environment, comprising a means to display data, means operable by a user to select desired data and activate the display means, and remote data store means adapted to provide data digitally to the display means when accessed by the user. Such data may comprise telephone messages, television or video pictures, radio or audio signals as well as textual information.

The user may be a patient or an authorised member of the hospital staff and the display means may comprise a visual display console, preferably a touch screen console or alternatively the console may be operable by a keyboard.

The keyboard may comprise a portable keyboard.

These alternatives are particularly simple to use by a patient in a bed in a hospital.

The console may be activated by the patient for entertainment and communication purposes by means of a password or pin number. Hospital staff may use the console to access the hospital data system by means of an electronic key or smart card means, preferably comprising an electronic USB smart key or smart card. This provides for dedicated secure use by authorised hospital staff, and is for clinical access only.

The hospital data may comprise medical data relating to a particular patient user of the system. Preferably the medical data may be part of the medical inventory of a hospital comprising said health care environment. This provides the advantage that a patient user can access his/her medical records as long as an authorised member of the hospital staff is present.

Access to the medical data of a particular patient may be limited to that patient or a doctor, paramedic or nurse caring for that patient.

There may be security means comprising an electronic fire-wall and security server.

There may be one or more servers, core switches of the entertainment and communication system, hospital core switches and one or more hospital servers, and the fire wall may be interposed between the system servers or core switches and the hospital servers or core switches.

The system core switches may be connected with the patient console by means of a fibre optic carrier, edge switches, and a client server.

The console server may comprise part of mounting means of the console.

The edge switches and patient console server may be connected by data cabling.

The mounting means of the console may comprise a bracket mountable on a substrate, for example a wall, such as a wall or ceiling of a hospital ward.

There may be a digital network which may comprise a data content delivery satellite system and/or a terrestrial data transmitter the or each of which is remote from a health care environment.

The system may have a head end which may have a server comprising a plurality of discrete data servers and another fire wall for protection from the worldwide web..

The discrete data servers may comprise a plurality of TV and/or radio servers.

The head end may be adapted to serve a plurality of floors in a health care environment such as a hospital.

The system may be adapted to provide access via the network to data for each individual patient in a particular bed in the hospital.

The network may comprise a wireless LAN to enable the use of portable data and communication devices. The wireless LAN may comprise an 802.11 wireless LAN.

The edge switches may provide 96 ports with a minimum of 10.2 Mbits per port.

The system may comprise an ADSL connection to each health care environment.

The system may comprise content streaming from the remote data store means.

The system may comprise remote monitoring and on-line support for the patient/user.

The system may also comprise a web hosting facility.

The console may be mounted on an elongate support means, which may preferably comprise a swivel means.

The swivel means may comprise an arm which is telescopic.

The swivel arm may mount the console for substantially 360° movement.

The bracket may contain a PC.

The system may comprise a single fast Ethernet network.

A system embodying the invention may be compatible with hospital data systems, and may suitably be modular. This provides for ease of installation in different size of health care environment.

The system may be digital and interactive at the console whereby to provide services to a patient/user, which services may comprise internet and e-mail services, and access to hospital data systems.

Using the invention it is thus possible to provide digitally a high resolution touch screen with say 1024 X 768 pixels, an intuitive interface with picture in picture functions, and a remote control of the data accessed on the console via an IP telephone. Each "host" or health care environment suitably has a broad band connection facility.

According to a further aspect, the invention provides a data display means, adapted to be mounted for viewing by a particular patient/user in a health care environment, and comprising mounting means including a PC for receiving data to be displayed on the data display means.

A system for providing services to a patient in a health care environment is hereinafter described, by way of example, with reference to the accompanying drawings.
Fig. 1 is a schematic view of a system according to the invention;
Fig. 2 is a schematic elevational view of a "screen" of a console of a system according to the invention;
Fig. 3 is a schematic elevational view of another "screen" of a console of a system according to the invention;
Fig. 4 is a schematic elevational view of another "screen" of a console of a system according to the invention;
Fig. 5 is a schematic view of a security set-up for a system according to the invention;
Fig. 6 is an elevational view to an enlarged scale of a patient bed-side arrangement of a system according to the invention;
Fig. 7 is a plan view of the arrangement of Fig. 7;
Fig. 8 is a perspective view of a support arm of the arrangement of Figs. 7 and 8 absent a console;

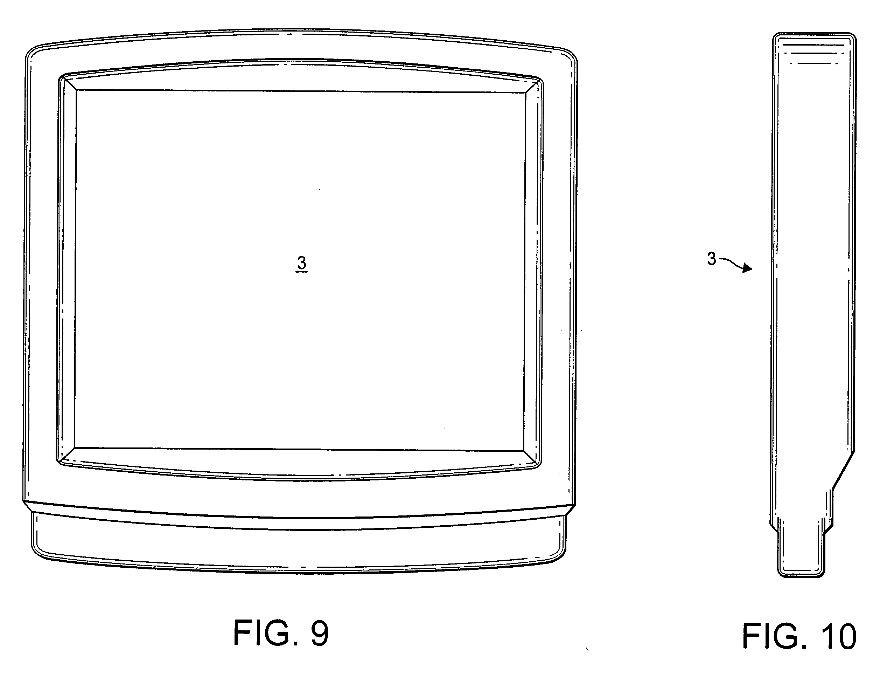

Figs. 9 and 10 are respective plan and side elevational views of a console for use in a system according to the invention; and
Figs. 11 and 12 are views similar to Figs, 10 and 11 of a touch pad control for use with a console of a system according to the invention.

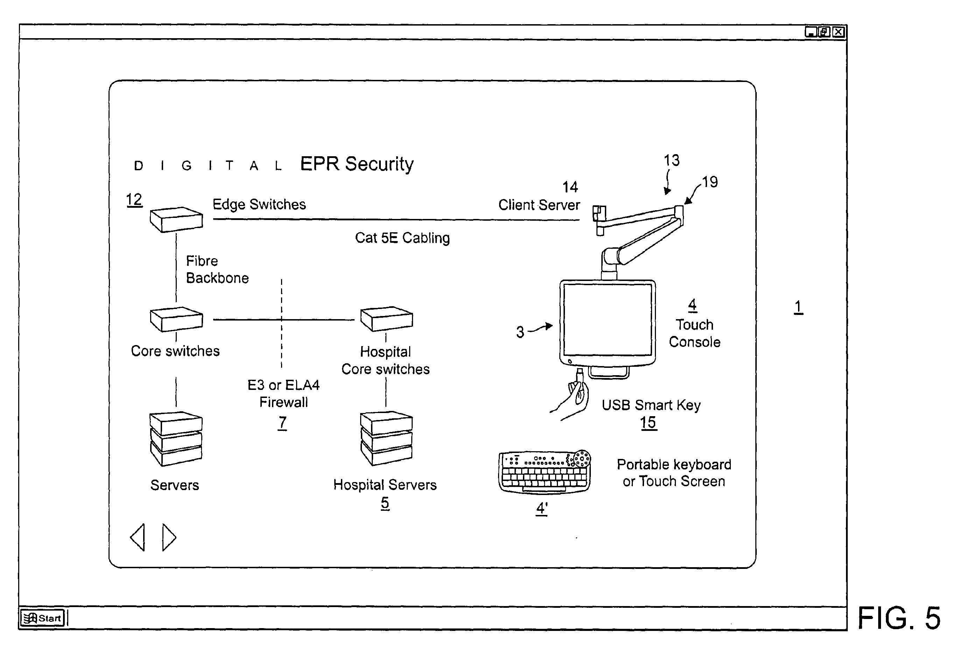

Referring to the drawings there is shown a system 1for providing services to a patient in a health care environment, such as a hospital 2, comprising a means 3 (Fig. 5) to display data, means 4, 4' operable by a user to select desired data and activate the display means 3, and remote data store means such as servers 5 (Fig. 5) adapted to provide data digitally to the display means 3 when accessed by the user. The overall system 1 as shown in the drawings can provide patient access to entertainment, communication, and medical data relating to a patient and held by or on a hospital data base. The medical condition of each patient, in other words an inventory of medical data is thus accessible.

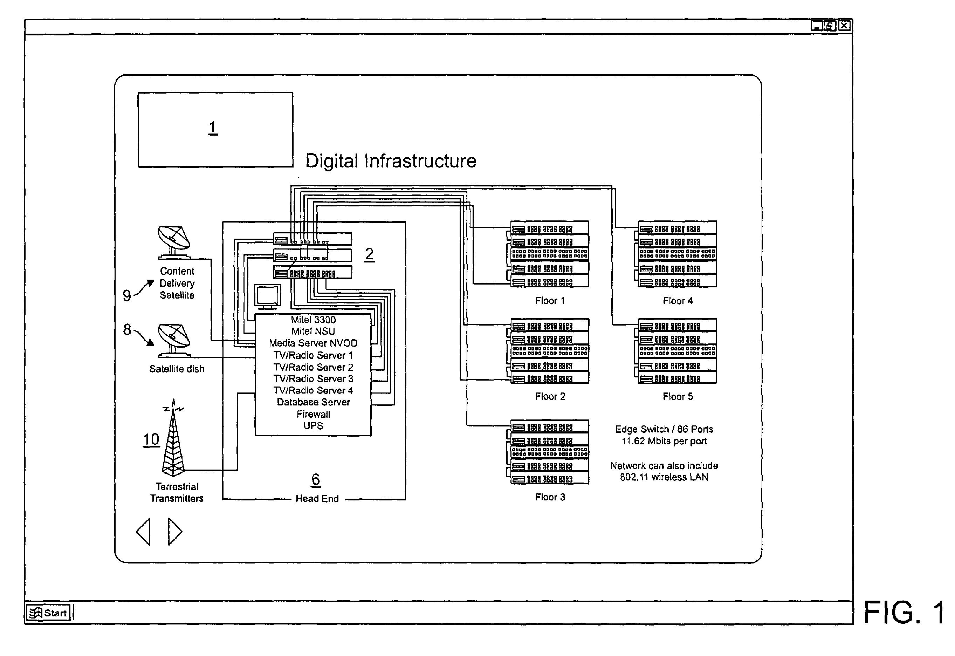

The hospital 2 shown in Fig. 1 has several locations such as floors, e.g. Floor 1, Floor 2, Floor 3, Floor 4 and Floor 5 which provide a digital infrastructure having at a head end 6 in other words at a bedside, the display means 3 comprising a console with a media server VOD/NVOD in the example a Mitel 3300 and Mitel NSU including a plurality of TV and radio servers, for an example, a data base server, an electronic fire wall 7 (Fig. 5) and UPS. The system infrastructure is served by a satellite dish 8 at the head end 6 receiving content delivery from a content delivery satellite 9 and/or terrestrial transmitters 10. At each location there is an edge switch 12 including in the embodiment 96 ports of 10.2 Mbits per port, and it will also be appreciated that the digital infrastructure providing the network disclosed can also include an 802.11 wireless LAN.

At the digital head end 6 of the system 1 or network there is provision for a plurality of racks for example two 30 inch (76.2cm) racks, a plurality of configurations for example three for small, medium and large patient care centres such as hospitals having up to 1500 beds. Moreover for a small hospital, for example with less than 150 beds, service and software streaming will be used, while for large hospitals for example of over 400 beds the network 1 provides for hardware appliances and streaming, while in any application there are extreme or other Ethernet switches and voice and voice mail solutions. The Mitel applications provides IP labelled switch reporting IP digital and analogue extensions which can operate with PSDN or ADSL networks having a compact size and rack mounting, can be commissioned and have long term support from global distributors and systems integrators, has inter operability with selected Ethernet switches, and a high level of support. This support for the system in the embodiment comprises an ADSL connection to each site, content streaming from a central source for example films, gaming, story books, audio books etc., remote monitoring and on-line support, web hosting, on-line assistance with network and systems design and long term maintenance and software management.

A system 1 embodying the invention provides that at each patient bed there is a data display means in the form of the touch screen monitor 3, suitably a 15 inch (38.1 cm) touch screen mounted on a support 13 such as a swivel arm which can be both telescopic and swivelable through 360° to provide for ergonometric user access. The touch screen monitor or console 3 is suitably mounted at one end of the arm 13, the other end of which is supported on a substrate such as a wall of a ward by a bracket which essentially comprises a wall box which houses the personal computer, operable via the single fast Ethernet network, it is compatible with the hospital data systems and thus provides a modular, industry standard architecture with fully digital interactive patient services including internet and e-mail as well as secure rapid access to hospital data. All these services including entertainment services and access to medical data are thus provided at the patient bedside and with suitable key systems such as USB smart keys 15, a system 1 can be essentially dedicated to a particular patient. The touch screen 3 itself is a high resolution touch screen for example a 1024 X 76 pixels has an intuitive interface with in picture functionality an IP telephone handset 16 which can also provide remote control, a wide range of digital interactive patient services, an integral patient billing system, 3 USB ports per terminal and access to hospital trust networks through password protected USB smart keys 15. This latter function can provide for the accessing to medical records of a patient held in a central hospital patient data base 5. The system 1 thus essentially provides for bedside computing which itself provides for access to electronic patient records, management of beds and patient identification, integration with patient administration systems including catering (the patient can order a meal) and dietary control, patient satisfaction surveys, digital imaging transmission to the bedside, health videos and websites for informed consent from a patient, patient monitoring, cognitive exercises and therapies, physiotherapy exercises and reminders for a patient, management of pain via patient controlled analgesia systems, nurse clerking and pre-op checks and finally can provide for administration of discharge of a patient and medication both during hospital stay and after discharge, when the patient returns home following treatment in a hospital.

In order for a patient to access the system, each patient has a personalised PIN number which is entered on the console 3 either remotely or by touch on a plurality of in the embodiment, six, numbers which when entered can provide a patient access to the full services provided by the system on "clicking" on a sign in button and moreover, there is also a facility for reminding a patient of his/her PIN number if that number is forgotten, by clicking on a button dedicated by the legend "forgotten your PIN?".

Once the PIN number has been correctly entered, a bar providing for options to be selected, for example various TV channels as well as a main menu button which will when operated by touch screen or remotely via the IP telephone handset provide all the systems available to the user/patient - it being understood that the user could be a nurse, doctor or relative directly at the bedside of the patient, in addition to the patient him/herself, the touch screen console or monitor 1 also providing for stop facilities, pause facilities, rewind, fast forward, volume control, contrast and a "Help" switch.

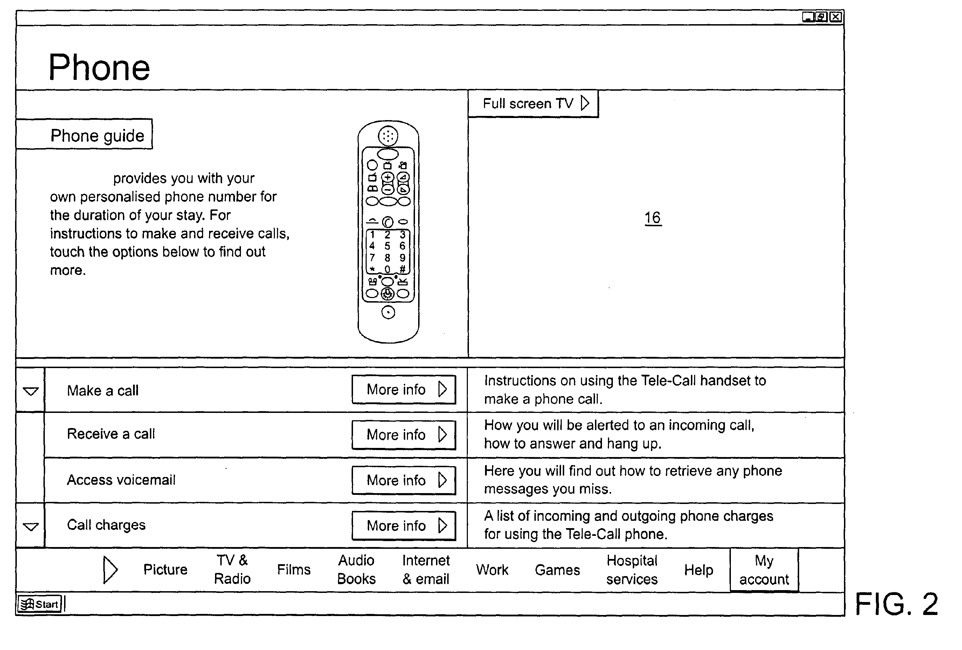

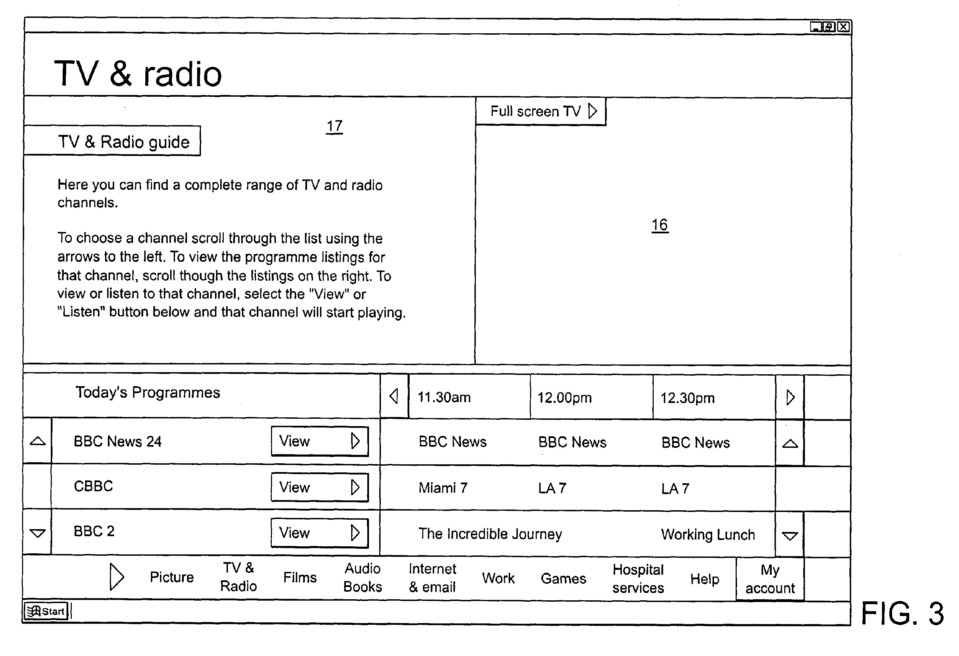

The system 1 also provides for in-screen screens, in other words a TV programme can occupy a part for example a quarter of the size of the screen of the console 1 (as shown at 16 in Figs. 2 and 3, but there is a facility for providing for full screen display.

Fig. 2 shows one example of a display screen which includes a bottom line of selections for phone, TV and radio, films, audio books, internet and e-mail with games and hospital services as well as help and "my account" buttons with simple touch instructions for example making a telephone call, receiving a telephone call, access to voice mail and call charges. As is seen from Fig. 2, while the phone facility is displayed it is also possible to have a TV programme displayed, thus providing multi-media access.

Reverting to the telephone facility, each items such as "make a call" has an additional touch screen entry button for more information for example providing instructions on using a hand set to make a telephone call.

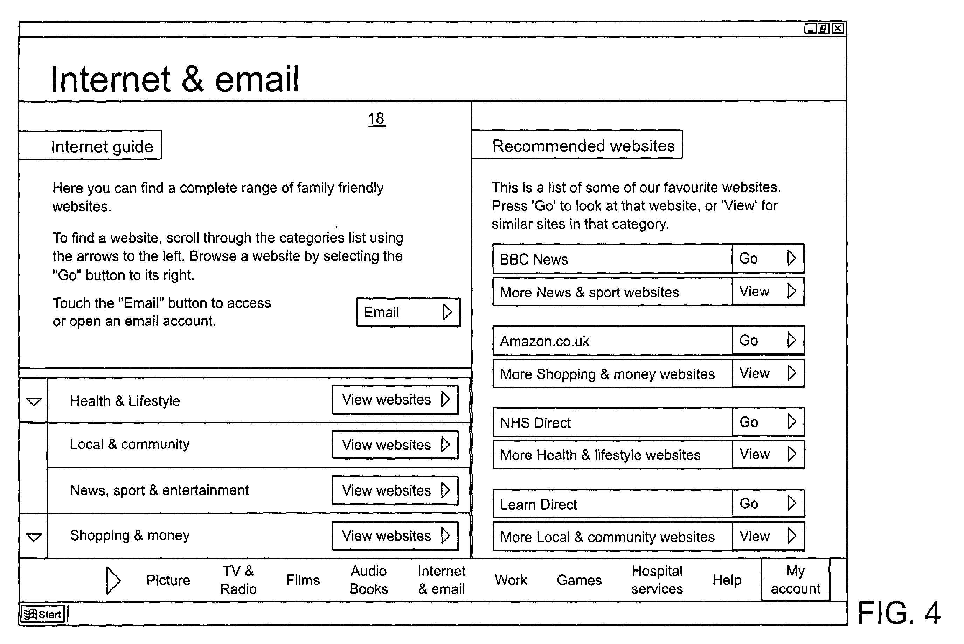

Fig. 3 shows a similar screen 17 to that of Fig. 2 but with actions for TV and radio programmes while, again, Fig. 4 shows another display 18 on a console of the system 1 for internet and e-mail use.

Fig. 5 is a schematic array showing the security arrangements previously referred to with the servers core switches with a fibre backbone, hospital core switches and hospital servers with a fire wall such as in E3 or EA4 standard between them, an edge switch connected to the wall mounted PC 14 of the touch console 3 with the USB smart key and the portable keyboard which can also be used to "touch" screen as seen mounted on the swivel arm via the wall mounted box including the client server/personal computer.

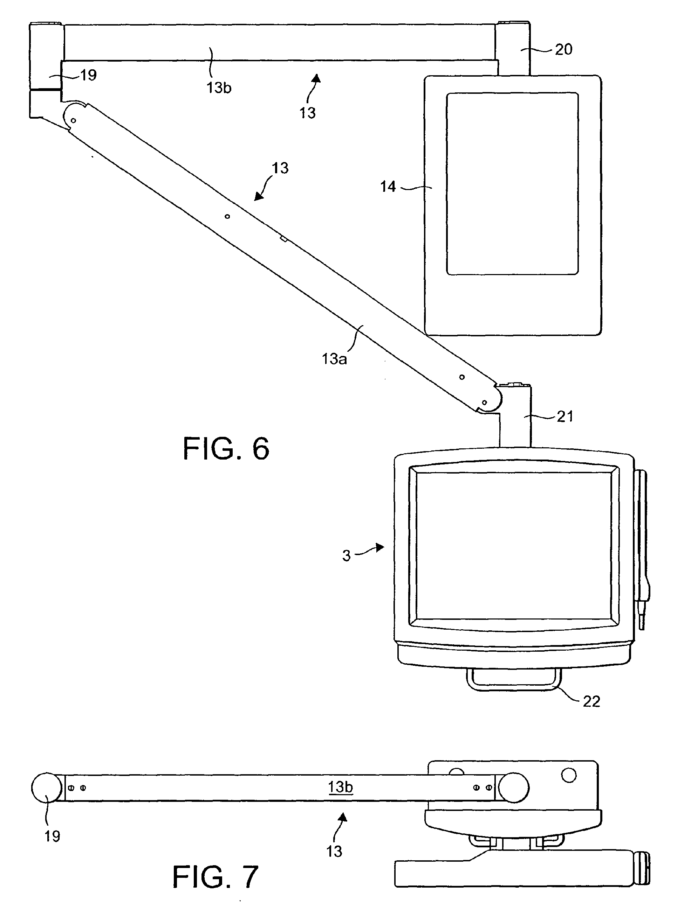

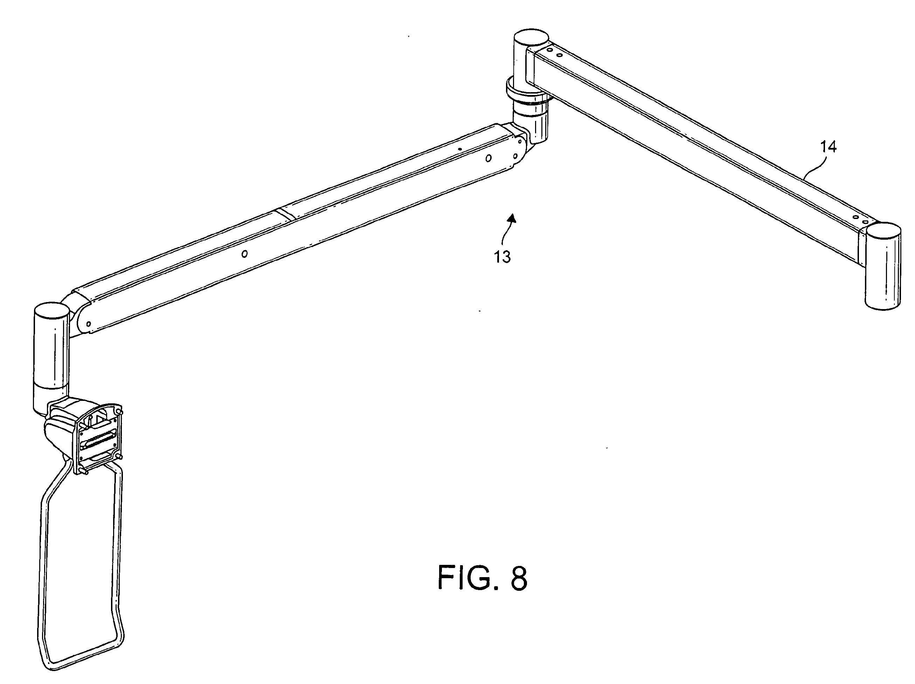

Figs. 6 and 7 show the display of the console mounted on the swivel arm 13 and connected to the wall bracket 14, the console being suspended for 360° of movement via the swivel arm which is in two parts 13a, 13b which are shown in Fig. 8, extending on intercepting orthogonal planes and being connected by a vertical, as viewed, pivot point 19 intermediate a vertical pivot point 20 connecting the arm 13a to the PC 14 and a vertical pivot 21 on which the console 3 is mounted at the end of the arm part 13a.

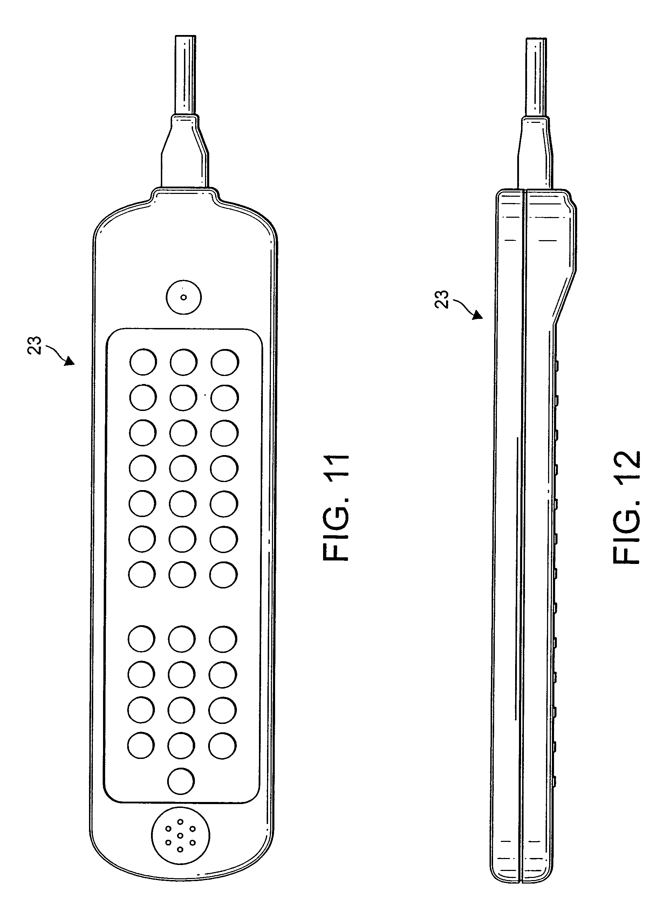

The console has a U-shaped handle 22 for ease of manipulation while Figs. 11 and 12 show a keypad 23 for keypad control and telephone operation.

It will be understood that using the invention it is possible to provide a single integrated record system including an electronic records system and for services to a patient/user at a bedside, the system being adapted digitally for dedicated use by a particular patient on demand.

## Claims

1. A digital system for providing services to a patient in a health care environment, **characterised by** a means to display data, means operable by a user to select desired data and activate the display means, and remote data store means adapted to provide data digitally to the display means when accessed by the user.

2. A system according to Claim 1, **characterised by** the user being a patient and the display means comprising a visual display console.

3. A system according to Claim 2, **characterised by** the console comprising a touch screen console.

4. A system according to Claim 2, **characterised by** the console being operable by a keyboard.

5. A system according to Claim 4, **characterised by** the keyboard comprising a portable keyboard.

6. A system according to any of Claims 2 to 5, **characterised by** the console being activated by an electronic key means.

7. A system according to Claim 6, **characterised by** the key means comprising an electronic USB smart key or smart card(s).

8. A system according to any preceding claim, **characterised by** the data comprising medical data relating to a particular patient user of the system.

9. A system according to Claim 8, **characterised by** the medical data being part of the medical inventory of a hospital comprising said health care environment.

10. A system according to Claim 9, **characterised by** access to the medical data of a particular patient being limited to that patient or a doctor, paramedic or nurse caring for that patient.

11. A system according to Claim 10, **characterised by** including security means comprising an electronic fire-wall and a security server.

12. A system according to Claim 11, **characterised by** one or more servers, core switches of the system, hospital core switches and one or more hospital servers, the fire wall being interposed between the or each system server and core switches and the hospital core switches and the or each hospital server.

13. A system according to Claim 12, **characterised by** the system core switches being in series with a fibre optic carrier, edge switches, and a server connected with the patient console.

14. A system according to Claim 13, **characterised by** the console server comprising part of mounting means of the console.

15. A system according to Claim 14, **characterised by** the edge switches and patient console server being connected by data cabling.

16. A system according to Claim 14 or Claim 15, **characterised by** the mounting means of the console comprising a bracket mountable on a substrate.

17. A system according to Claim 16, **characterised by** the substrate comprising a wall or ceiling.

18. A system according to any of Claims 10 to 17, **characterised by** a digital network comprising a data content delivery satellite system and/or a terrestrial data transmitter the or each of which is remote from a health care environment.

19. A system according to Claim 18, **characterised by** there being a head end having a server comprising a plurality of discrete data servers and the fire wall.

20. A system according to Claim 19, **characterised by** the discrete data servers comprising a plurality of TV and/or radio servers.

21. A system according to Claim 20, **characterised by** the head end being adapted to serve a plurality of floors in a health care environment such as a hospital.

22. A system according to Claim 21, **characterised by** adapted to provide access via the network to data for each individual patient in a particular bed in the hospital.

23. A system according to Claim 22, **characterised by** the network comprising edge switches and/or wireless LAN per USB port.

24. A system according to Claim 23, **characterised by** the edge switches providing 96 ports with 10.2 Mbits per port.

25. A system according to Claim 23 or Claim 24, **characterised by** the wireless LAN comprising an 802.11 wireless LAN.

26. A system according to any of Claims 10 to 26, **characterised by** comprising an ADSL connection to each health care environment.

27. A system according to any of the preceding claims, **characterised by** content streaming from the remote data store means.

28. A system according to Claim 27, **characterised by** remote monitoring and on-line support for the patient/user.

29. A system according to Claim 28, **characterised by** comprising a web hosting facility.

30. A system according to any of Claims 14 to 29, **characterised by** the console being mounted on an elongate support means.

31. A system according to Claim 30, **characterised by** the elongate support means comprising a swivel means.

32. A system according to Claim 31, **characterised by** the swivel means comprising an arm which is telescopic.

33. A system according to Claim 32, **characterised by** the swivel arm mounting the console for substantially 360° movement.

34. A system according to any of Claims 10 to 33, **characterised by** the bracket comprising a PC.

35. A system according to any of Claims 10 to 34, **characterised by** comprising a single fast Ethernet network.

36. A system according to Claim 35, **characterised by** being compatible with hospital data systems.

37. A system according to Claim 36, **characterised by** being modular.

38. A system according to any of Claims 10 to 37, **characterised by** being digital and interactive at the console whereby to provide services to a patient/user.

39. A system according to Claim 38, **characterised by** said services comprising internet and e-mail services.

40. A system according to Claim 39, **characterised by** the services comprising access to hospital data systems.

41. A system according to any preceding claim, **characterised by** comprising an integral patient billing system for paying for multimedia entertainment and/or telephony services of the system.

42. A data display means, adapted to be mounted for viewing by a particular patient/user in a health care environment, and comprising mounting means including a PC for receiving data to be displayed on the data display means.

43. A data display means according to Claim 42, **characterised by** comprising a console mounted and the mounting means having an arm projecting from a bracket housing the PC.

44. A data display means according to Claim 43, **characterised by** the arm being adjustable.
